# EUROPEAN PATENT SPECIFICATION

(11) **EP 0 962 191 B1**
(45) Date of publication and mention of the grant of the patent: **09.05.2007**
(21) Application number: 99304331.4
(22) Date of filing: 03.06.1999
(51) Int. Cl.: A61B 18/14, A61B 18/24

(54) **Catheter for injecting therapeutic and diagnostic agents**
KATHETER ZUM INJIZIEREN THERAPEUTISCHER UND DIAGNOSTISCHER MITTEL
Catheter pour injecter des agents therapeutiques et diagnostiques

(30) Priority: 04.06.1998 US 88019 P; 11.06.1998 US 88984 P; 29.03.1999 US 280202
(43) Date of publication of application: 08.12.1999
(73) Proprietor: Biosense Webster, Inc., Baldwin Park, CA 91706 (US)
(72) Inventor: Hill, Irma P., Glendora, California 91741 (US); Ponzi, Dean M., Glendora, California 91741 (US)
(74) Representative: Mercer, Christopher Paul

(56) References cited:
- WO-A-96/41654
- US-A- 5 403 311
- US-A- 5 431 168
- US-A- 5 522 815
- US-A- 5 588 432

## Description

### FIELD OF THE INVENTION

This invention relates to a catheter for infusing therapeutic or diagnostic agents into the tissue of organs, and more particularly to a catheter system in which the infusion needle may be very precisely positioned within the heart to infuse drugs into the wall of the heart.

### BACKGROUND OF THE INVENTION

Targeted delivery of therapeutic or diagnostic agents, such as occurs in gene therapy, is very desirable but often presents a difficult challenge. A potential benefit of targeted delivery is that there is an increased efficiency obtained by the precise placement of the therapeutic agent. There are several problems to this procedure which must be overcome in order to obtain satisfactory results from such therapy, such as the problems of obtaining access to the delivery site, transporting the therapeutic agent to the desired site, injecting the therapeutic agent at the proper depth within the organ tissue, steering the distal end of the catheter to a desired location within the organ prior to infusing the agent, and positioning the distal tip of the catheter at precisely the same location where prior measurements have indicated that the drug should be infused. It is also important for a physician to be able to monitor the position of the infusion needle with respect to the wall of the organ. In the case of an organ, such as the heart, in which the walls are in constant motion, the activity of positioning and monitoring the position of the distal tip of the catheter, or infusion needle, becomes especially difficult.

U.S. Patent No. 3,598,119 discloses a medical device for injecting drugs in which the injection needle is guided through an inner lumen of a catheter for insertion of the needle under skin tissue. A bladder at the distal end of the catheter may be inflated through another lumen for holding the point of the needle point in a fixed position beneath the skin.

U.S. Patent No. 4,578,061 discloses a catheter for injecting a liquid into a vein, or artery, through an injection needle which is longitudinally movable beyond the distal end of the catheter. A dual chamber system is utilized within the catheter tip to provide for movement of a plunger to extend the injection needle and also to allow for a plunger to be used to apply a predetermined dose of medication through the injection needle.

U.S. Patent No. 4,578,061 discloses an injection catheter having a longitudinal movable needle which may be moved through a lumen in order to extend out of the side wall of the catheter for injecting a liquid into a blood vessel. The needle is normally retracted into the device so that the needle will not penetrate tissue as the device is moved through a body duct. Thereafter, the needle is moved out of the side of the catheter into a vessel wall in order to infuse a liquid into the wall of a vessel.

U.S. Patent No. 5,244,460 is directed toward a method for improving blood flow to the heart. More particularly this patent is directed toward a medical procedure for improving the growth of cardiac blood vessels by inserting a catheter into a coronary artery and injecting into the heart a blood vessel growth promoting peptide through an injection port of the catheter.

U.S. Patent No. 5,419,777 is directed toward a catheter for injection of a fluid into body cavities such as coronary vessels and arteries. This patent, as is the case with the '061 patent illustrates the use of an injection needle which protrude laterally through the side walls of the distal tip of the catheter. In the case of drug injections to be made into coronary vessels and arteries, it is very desirable to have the needles extend out of the side walls of the catheter and at an acute angle to the walls of the vessel in order to penetrate the walls of the vessel for injection of the agent.

U.S. Patent 5,431,168, assigned to the same assignee as the present patent application, is directed toward a steerable catheter which includes a puller wire for controlling the distal end of the catheter from a control handle which is mounted on the proximal end of the catheter.

Copending U.S. Patent Application Serial No. 09/019,453, entitled "Intracardiac Drug Delivery," assigned to an affiliated company of the assignee of this application, (now published as US 6 309 370) discloses an injection catheter system for infusing a diagnostic or therapeutic agent into the wall of an organ which includes an electromagnetic sensor disposed within the distal tip of the catheter for providing very precise location information for the distal tip of the catheter.

US 5,588,432 discloses a steerable acoustical imaging catheter having an ultrasound device and an electrode mounted on the catheter to create an image of an internal structure and to make electrical contact therewith respectively. A chemical ablation device is also mounted on the catheter to ablate a portion of the internal structure by the delivery of fluid.

Document US-A-5 403 311 discloses a steerable cardiac drug injection catheter comprising: a deflection control handle, a tip section having on its distal end a tip electrode and a lumen therethrough, an electrode conductor, an injection needle being slidable between a first position, in which the distal tip of the needle is withdrawn into the distal face of the tip electrode, and a second position, in which the distal tip of the needle extends out of the distal face of the electrode, a needle control handle and means for deflecting the distal tip of the catheter upon manipulation of the deflection control handle.

For obvious reasons, catheters which are used to deliver therapeutic or diagnostic agents into a targeted region of the heart must be designed so that the physician is able to maintain precise control over the distal tip of the catheter. In addition, the catheter must be designed to provide information as to the precise location of the distal tip, or infusion needle, of the catheter. The present invention is directed toward an improved injection catheter which allows the physician to have greater control over the position of the distal tip and to also obtain accurate information as to the position of the catheter tip.

### SUMMARY OF THE INVENTION

According to the present invention, there is provided a steerable cardiac drug injection catheter comprising:
a catheter body having an outer wall proximal and distal ends, and at least one lumen extending therethrough;
a deflection control handle fixedly attached to the proximal end of the catheter body;
a tip section comprising flexible tubing having proximal and distal ends and at least one lumen extending therethrough, the proximal end of the tip section being fixedly attached to the distal end of the catheter body;
a tip electrode mounted at the distal end of the tip section said tip electrode having a distal face and a lumen therethrough and being adapted to measure electrical potentials;
an electrode conductor electrically connected to the tip electrode, said conductor extending through the at least one lumen in the tip section, through the at least one lumen in the catheter body and into the deflection control handle;
an injection needle extending through the at least one lumen in the catheter body and the at least one lumen in the tip section and through the lumen of the tip electrode, said needle being slidable from a first position in which the distal tip of the needle is withdrawn into the distal face of the tip electrode to a second position in which the distal tip of the needle extends out of the distal face of the tip electrode;
a needle control handle including a needle control knob attached to a piston which is slidably mounted within a lumen within an outer body of the needle control handle, the injection needle being fixedly attached to the outer body and extending through the lumen within the outer body and through a lumen in the piston, the needle control handle arrangement being for sliding the needle from the first position to the second position; and
means for deflecting the distal tip of the catheter upon manipulation of the deflection control handle.

This tip electrode is used for measuring electrical potentials within the heart, for example cardiac mapping.

In accordance with another aspect of the present invention, the drug injection catheter includes an electromagnetic mapping sensor disposed in the distal portion of the tip section for producing electrical signals indicative of the location of the electromagnetic mapping sensor relative to the position of a reference. This provides very precise information with respect to the location of the distal tip of the catheter.

In accordance with still another aspect of the present invention, the drug injection catheter includes a sensor cable electrically attached to the electromagnetic mapping sensor and extending through the at least one lumen in the tip section, through the at least one lumen of the catheter body and into the deflection control handle where it is then electrically attached to a circuit board situated within the deflection control handle.

This provides precise information as to the location of the distal tip of the catheter.

In accordance with still another aspect of the present invention, the deflection control handle comprises a first member fixedly attached to the proximal end of the catheter body and a second member which is movable with respect to the first member. The catheter comprises a puller wire having a proximal end and a distal end which extends from the defection control handle through the at least one lumen in the catheter body and is fixedly secured within the tip section. The proximal end of the puller wire is fixedly secured to the second member of the deflection control handle whereby manipulation of the first member of the handle relative to the second member of the deflection control handle moves the puller wire relative to the catheter body resulting in deflection of the tip section.

In accordance with still another aspect of the present invention, the first member of the deflection control handle is movable from a first position to a second position relative to the second member of the deflection control handle, and the puller wire is fixedly secured to the second member of the deflection control handle such that when the second member of the deflection control handle is moved from the first position to the second position the puller wire causes the tip section of the catheter to be deflected from a normally straight position to a deflected position.

In accordance with still another aspect of the present invention, the deflecting control further comprises a compression coil situated in the catheter body in surrounding relation to the puller wire and extending into the at least one lumen in the tip section. This prevents buckling of the catheter shaft. The compression coil is anchored to the catheter at the proximal end of the catheter body and at the proximal end of the tip section.

In accordance with still another aspect of the invention, the outer diameter of the needle is substantially the same as the diameter of the at least one lumen through the tip section to thereby prevent the flow of blood through the lumen of the tip section and the at least one lumen of the catheter body.

### BRIEF DESCRIPTION OF THE DRAWINGS

These and other features and advantages of the present invention will be better understood by reference to the following detailed description when considered in conjunction with the accompanying drawings wherein:
Figure 1 is a side plan view of one embodiment of the catheter of the present invention;
Figure 2 is a side cross-sectional view of the needle control handle for the embodiment of Figure 1;
Figure 3 is a side cross-sectional view of the catheter tip section showing an embodiment having three lumens and showing the position of the electromagnetic mapping sensor and the injection needle;
Figure 4 is a side cross-sectional view of the catheter tip section showing an embodiment having three lumens and showing the position of the electromagnetic mapping sensor and the puller wire;
Figure 5 is a side cross-sectional view of the catheter body, including the junction between the catheter body and the tip section;
Figure 6 is a transverse cross-sectional view of the catheter tip section along line 6-6 showing an embodiment having three lumens;
Figure 7 is a transverse cross-sectional view of the catheter body along line 7-7; and,

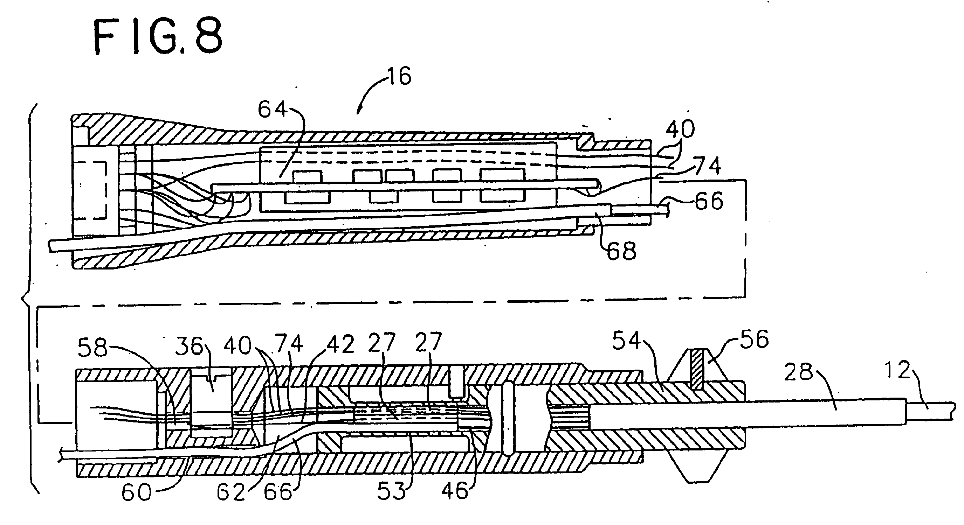

Figure 8 is a side cross-sectional view of the catheter handle;

### DESCRIPTION OF A PREFERRED EMBODIMENT

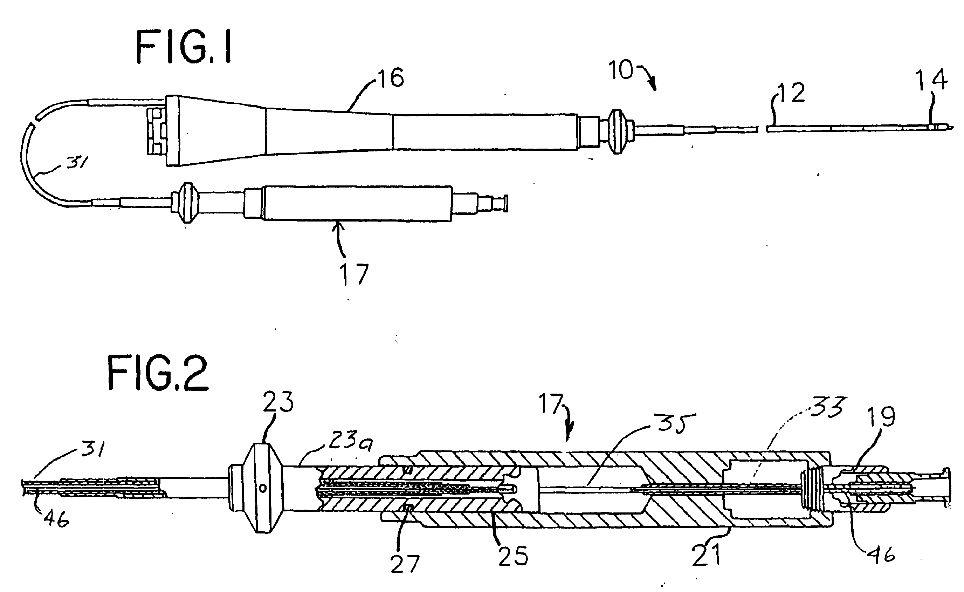

In a preferred embodiment of the invention, there is provided a catheter for use for injection of a therapeutic or diagnostic agent into the heart. As shown in Figure 1, catheter 10 is comprised of an elongated catheter body 12 having proximal and distal ends, a tip section 14 at the distal end of the catheter body 12, and a deflection control handle 16 at the proximal end of the catheter body 12 and a needle control handle 17.

As illustrated in Figure 2, the needle control handle 17 is comprised of a proximal Luer connector 19 which is threaded into an outer body 21. The control handle also includes a slidable control knob 23 which is attached to a piston 23a, which is in turn slidably mounted within a lumen 25 within the outer body 21. A ring seal 27 is disposed between the piston 23a and the inner lumen of the outer body 21 to prevent fluid from entering the housing. The slidable control knob 23 and piston 23a are fixedly attached to a catheter tubing 31 which extends into the needle control housing 17 and the deflection control housing 16.

In addition, the needle control housing 17 includes a support tube 33 which is fixedly mounted coaxially within the outer body 21 and is preferably formed of stainless steel. Positioned within the lumen of support tube 33 is a slidable tube 35, preferably formed of stainless steel, which is in turn directly coupled to the proximal end of the catheter tubing 31. An injection needle 46 is fixedly attached to an inner lumen of the Luer connector 19 and extends through the lumen of the slidable tube 35 and then through a lumen in the slidable piston 23a and then into the catheter tubing 31.

Accordingly, since the injection needle is attached to the Luer connector 19, it may be seen that as the control knob 23 is moved proximally to thereby cause the piston 23a to move proximally into the outer body 21 of the needle control housing, the injection needle 46 is caused to slide through the catheter housing 31 with the result that the needle is caused to be extended out of the distal end of the injection catheter system 10. Also, it may be seen that as fluid is applied to the inner lumen of the Luer connector, the fluid is caused to flow through the lumen of the injection needle and to the distal tip of the injection needle 46.

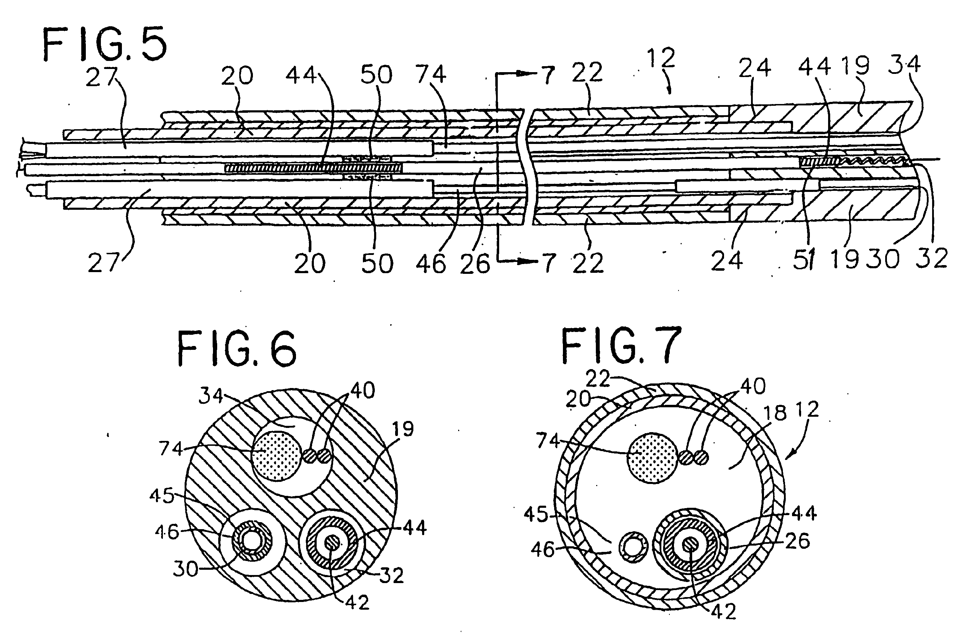

With reference to Figures 5 and 7, the catheter body 12 comprises a single, central or axial lumen 18. The catheter body 12 is flexible, i.e., bendable, but substantially non-compressible along its length. The catheter body 12 may be of any suitable construction and made of any suitable material. A presently preferred construction comprises an outer wall 22 made of a polyurethane or nylon. The outer wall 22 comprises an imbedded braided mesh of stainless steel or the like to increase torsional stiffness of the catheter body 12 so that, when the control handle 16 is rotated, the tip section of the catheter 10 will rotate in a corresponding manner.

The outer diameter of the catheter body 12 is not critical, but is preferably no more than about 2.67mm (8 French). Likewise the thickness of the outer wall 22 is not critical. The inner surface of the outer wall 22 is lined with a stiffening tube 20, which can be made of any suitable material, preferably polyimide. The stiffening tube, along with the braided outer wall 22, provides improved torsional stability while at the same time minimizing the wall thickness of the catheter, thus maximizing the diameter of the single lumen. The outer diameter of the stiffening tube 20 is about the same as or slightly smaller than the inner diameter of the outer wall 22. Polyimide tubing is presently preferred because it may be very thin walled while still providing very good stiffness. This maximizes the diameter of the central lumen 18 without sacrificing strength and stiffness. Polyimide material is typically not used for stiffening tubes because of its tendency to kink when bent. However, it has been found that, in combination with an outer wall 22 of polyurethane, nylon or other similar material, particularly having a stainless steel braided mesh, the tendency for the polyimide stiffening tube 20 to kink when bent is essentially eliminated with respect to the applications for which the catheter is used.

A particularly preferred catheter has an outer wall 22 with an outer diameter of about 2.34nm (0.092 inch) and an inner diameter of about 1.60mm (0.063 inch) and a polyimide stiffening tube having an outer diameter of about 1.56 mm (0.0615 inch) and an inner diameter of about 1.32mm (0.052 inch).

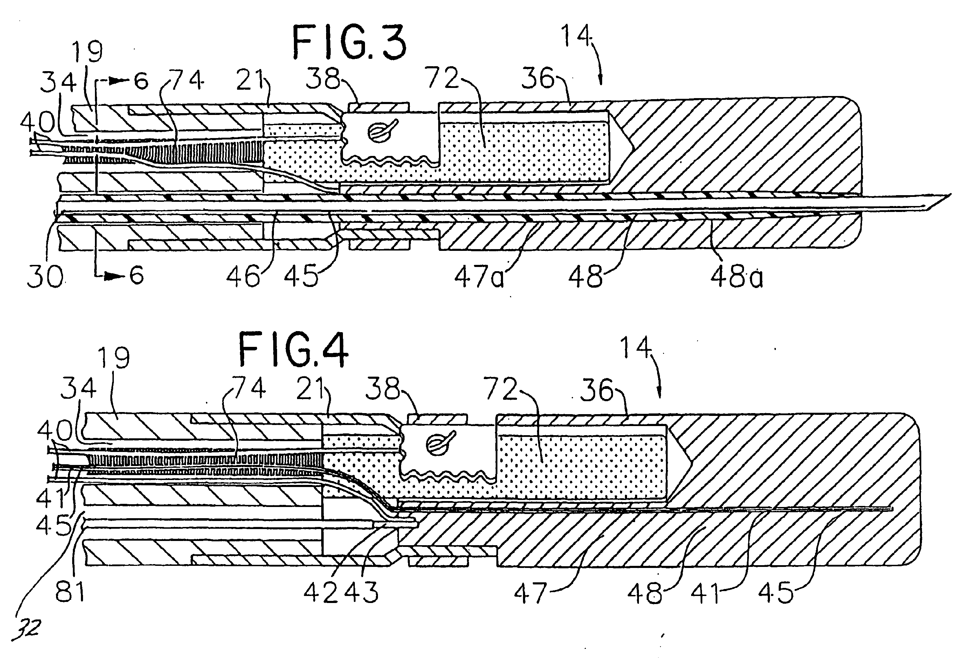

As shown in Figures 3 and 4, the tip section 14 comprises a short section of tubing 19 having three lumens. The tubing 19 is made of a suitable non-toxic material which is preferably more flexible than the catheter body 12. A presently preferred material for the tubing 19 is braided polyurethane, i.e., polyurethane with an embedded mesh of braided stainless steel or the like. The outer diameter of the tip section 14, like that of the catheter body 12, is preferably no greater than about 2.67 mm (8 French). The size of the lumens is not critical. In a particularly preferred embodiment, the tip section has an outer diameter of about 2.33 mm (7 French (.092 inch)) and the first lumen 30 and second lumen 32 are generally about the same size, having a diameter of about 0.56mm (0.022 inch), with the third lumen 34 having a slightly larger diameter of about 0.91mm (0.036 inch).

A preferred means for attaching the catheter body 12 to the tip section 14 is illustrated in Figure 5. The proximal end of the tip section 14 comprises an inner counter bore 24 that receives the outer surface of the polyimide stiffener 20. The tip section 14 and catheter body 12 are attached by glue or the like.

The stiffening tube 20 is held in place relative to the outer wall 22 at the proximal end of the catheter body 12. In a preferred construction of the catheter body 12, a force is applied to the proximal end of the stiffening tube 20 which causes the distal end of the stiffening tube 20 to firmly push against the counter bore 24. While under compression, a first glue joint is made between the stiffening tube 20 and the outer wall 22 by a fast drying glue, e.g. Super Glue®. Thereafter a second glue joint is formed between the proximal ends of the stiffening tube 20 and outer wall 22 using a slower drying but stronger glue, e.g., polyurethane.

Extending through the single lumen 18 of the catheter body 12 are lead wires 40, an injection needle 46, a sensor cable 74, and a compression coil 44 through which a puller wire 42 extends. A single lumen 18 catheter body is preferred over a multi-lumen body because it has been found that the single lumen 18 body permits better tip control when rotating the catheter 10. The single lumen 18 permits the lead wires 40, the injection needle 46, the sensor cable 74, and the puller wire 42 surrounded by the compression coil 44 to float freely within the catheter body. If such wires and cables were restricted within multiple lumens, they tend to build up energy when the handle 16 is rotated, resulting in the catheter body 12 having a tendency to rotate back if, for example, the handle is released, or if bent around a curve, to flip over, either of which are undesirable performance characteristics.

The puller wire 42 is anchored at its proximal end to the control handle 16 and anchored at its distal end to the tip section 14. The puller wire 42 is made of any suitable metal, such as stainless steel or Nitinol, and is preferably coated with Teflon® or the like. The coating imparts lubricity to the puller wire 42. The puller wire 42 preferably has a diameter ranging from about 0.15mm (0.006 inches) to about 0.25mm (0.010 inches).

The compression coil 44 extends from the proximal end of the catheter body 12 to the proximal end of the tip section 14. The compression coil 44 is made of any suitable metal, preferably stainless steel. The compression coil 44 is tightly wound on itself to provide flexibility, i.e., bending, but to resist compression. The inner diameter of the compression coil 44 is preferably slightly larger than the diameter of the puller wire 42. For example, when the puller wire 42 has a diameter of about 0.18mm (0.007 inches), the compression coil 44 preferably has an inner diameter of about 0.20mm (0.008 inches). The Teflon® coating on the puller wire 42 allows it to slide freely within the compression coil 44. Along its length, the outer surface of the compression coil 44 is covered by a flexible, non-conductive sheath 26 to prevent contact between the compression coil 44 and any of the lead wires 40, injection needle 46 or sensor cable 74. A non-conductive sheath 26 made of polyimide tubing is presently preferred.

The compression coil 44 is anchored at its proximal end to the proximal end of the stiffening tube 20 in the catheter body 12 by glue and at its distal end to the tip section 14. The glue may be applied by means of a syringe or the like through a hole made between the outer surface of the catheter body 12 and the single lumen 18.

The puller wire 42 extends into the second lumen 32 of the tip section 14. The puller wire 42 is anchored to a tip electrode 36 or to the side of the catheter tip section 14. With reference to Figures 4 and 5, within the tip section 14, and distal to the glue joint 51, the turns of the compression coil are expanded longitudinally. Such expanded turns 47 are both bendable and compressible and preferably extend for a length of about 12.7mm (0.5 inch). The puller wire 42 extends through the expanded turns 47 then into a plastic, preferably Teflon®, sheath 81, which prevents the puller 42 from cutting into the wall of the tip section 14 when the tip section 14 is deflected.

With reference to Figures 3 and 4, at the distal end of the tip section 14 is a tip electrode 36. Preferably the tip electrode 36 has a diameter about the same as the outer diameter of the tubing 19. The tip electrode 36 is connected to the tubing 19 by means of a plastic housing 21, preferably made of polyetheretherketone (PEEK). The proximal end of the tip electrode 36 is notched circumferentially and fits inside the distal end of the plastic housing 21 and is bonded to the housing 21 by polyurethane glue or the like. The proximal end of the plastic housing 21 is bonded with polyurethane glue or the like to the distal end of the tubing 19 of the tip section 14.

Mounted on the distal end of the plastic housing 21 is a ring electrode 38. The ring electrode 38 is slid over the plastic housing 21 and fixed in place by glue or the like. If desired, additional ring electrodes may be used and can be positioned over the plastic housing 21 or over the flexible tubing 19 of the tip section 14.

The tip electrode 36 and ring electrode 38 are each connected to separate lead wires 40. The lead wires 40 extend through the third lumen 34 of tip section 14, the catheter body 12, and the control handle 16, and terminate at their proximal end in an input jack (not shown) that may be plugged into an appropriate monitor (not shown). If desired, the portion of the lead wires 40 extending through the catheter body 12, control handle 16 and proximal end of the tip section 14 may be enclosed or bundled within a protective tube or sheath.

The lead wires 40 are attached to the tip electrode 36 and ring electrode 38 by any conventional technique. Connection of lead wire 40 to the tip electrode 36 is preferably accomplished by weld 43, as shown in Figure 4.

The injection needle assembly is comprised of the injection needle 46 which extends from the needle control handle through the body of the catheter, through the distal tip of the catheter and through the tip electrode 36. The injection needle 46 is formed of nitinol, and as illustrated in Figure 3 is preferably formed with a beveled edge at the distal tip of the needle. Also as illustrated in Figure 3, the needle is coaxially mounted within a polyimide tube 47a which serves to prevent the needle from buckling and also serves to electrically insulate the needle from the distal electrode 36. The tube 47a additionally serves to provide a fluid-tight seal surrounding the injection needle. The injection needle as shown in Figure 3 is in a position where the needle extends beyond the distal tip of the electrode as it would be positioned in order to infuse diagnostic or therapeutic fluid into the human heart. The needle is withdrawn within the distal tip of the catheter during the period of time that the catheter is inserted through the vasculature of the body and also during the period of time in which the catheter is removed from the body.

An electromagnetic sensor 72 is contained within the distal end of the tip section 14. The electromagnetic sensor 72 is connected by means of electromagnetic sensor cable 74, which extends through the third lumen 34 of the tip section 14 through the catheter body 12 into the control handle 16. The electromagnetic sensor cable 74 comprises multiple wires encased within a plastic sheath. In the control handle 16, the wires of the sensor cable 74 are connected to a circuit board 64. The circuit board 64 amplifies the signal received from the electromagnetic sensor and transmits it to a computer in a form understandable by the computer. Also, because the catheter is designed for single use only, the circuit board contains an EPROM chip which shuts down the circuit board after the catheter has been used. This prevents the catheter, or at least the electromagnetic sensor, from being used twice. A suitable electromagnetic sensor is described, for example, in U.S. Patent No. 4,391,199. A preferred electromagnetic mapping sensor 72 is manufactured by Biosense Ltd. Israel and marketed under the trade designation NOGA. To use the electromagnetic sensor 72, the patient is placed in a magnetic field generated, for example, by situating under the patient a pad containing coils for generating a magnetic field. A reference electromagnetic sensor is fixed relative to the patient, e.g., taped to the patient's back, and the injection catheter containing a second electromagnetic sensor is advanced into the patient's heart. Each sensor comprises three small coils which in the magnetic field generate weak electrical signals indicative of their position in the magnetic field. Signals generated by both the fixed reference sensor and the second sensor in the heart are amplified and transmitted to a computer which analyzes the signals and then displays the signals on a monitor. By this method, the precise location of the sensor in the catheter relative to the reference sensor can be ascertained and visually displayed. The sensor can also detect displacement of the catheter that is caused by contraction of the heart muscle.

Using this technology, the physician can visually map a heart chamber. This mapping is done by advancing the catheter tip into a heart chamber until contact is made with the heart wall. This position is recorded and saved. The catheter tip is then moved to another position in contact with the heart wall and again the position is recorded and saved.

The electromagnetic mapping sensor 72 can be used alone or more preferably in combination with the tip electrode 36 and ring electrode 38. By combining the electromagnetic sensor 72 and electrodes 36 and 38, a physician can simultaneously map the contours or shape of the heart chamber, the electrical activity of the heart, and the extent of displacement of the catheter and hence identify the presence and location of the ischemic tissue. Specifically, the electromagnetic mapping sensor 72 is used to monitor the precise location of the tip electrode in the heart and the extent of catheter displacement. The tip electrode 36 and ring electrode 38 are used to monitor the strength of the electrical signals at that location. Healthy heart tissue is identified by strong electrical signals in combination with strong displacement. Dead or diseased heart tissue is identified by weak electrical signals in combination with dysfunctional displacement, i.e., displacement in a direction opposite that of healthy tissue. Ischemic, or hibernating or stunned, heart tissue is identified by strong electrical signals in combination with impaired displacement. Hence, the combination of the electromagnetic mapping sensor 72 and tip and ring electrodes 36 and 38 is used as a diagnostic catheter to determine whether and where to infuse a drug into the wall of the heart. Once the presence and location of ischemic tissue has been identified, the injection catheter can be deflected so that the needle is normal, i.e., at a right angle, to the ischemic tissue, and the injection needle may then be moved out of the distal end of the catheter and into the wall of the heart.

It is understood that, while it is preferred to include both electrophysiology electrodes and an electromagnetic sensor in the catheter tip, it is not necessary to include both. For example, an injection catheter having an electromagnetic sensor but no electrophysiology electrodes may be used in combination with a separate mapping catheter system. A preferred mapping system includes a catheter comprising multiple electrodes and an electromagnetic sensor, such as the NOGA-STAR catheter marketed by Cordis Webster, Inc., and means for monitoring and displaying the signals received from the electrodes and electromagnetic sensor, such as the Biosense-NOGA system, also marketed by Cordis Webster, Inc.

The electrode lead wires 40, injection needle 46 and electromagnetic sensor cable 74 must be allowed some longitudinal movement within the catheter body so that they do not break when the tip section 14 is deflected. To provide for such lengthwise movement, there are provided tunnels through the glue joint 50, which fixes the proximal end of the compression coil 44 inside the catheter body 12. The tunnels are formed by transfer tubes 27, preferably made of short segments of polyimide tubing. In the embodiment shown in Figure 5, there are two transfer tubes 27 for the glue joint 50. Each transfer tube is approximately 60 mm long and has an outer diameter of about 0.53mm (.021 inch) and an inner diameter of about 0.48mm (.019 inch). Extending through one transfer tube 27 are the lead wires 40 and the electromagnetic sensor cable 74. Extending through the other transfer tube 27 is the injection needle 46.

An additional transfer tube 29 is located at the joint between the tip section 14 and the catheter body 12. Extending through this transfer tube is the injection needle 46. This transfer tube 29 provides a tunnel through the glue joint formed when the tip section 14 is glued to the catheter body 12. It is understood that the number of transfer tubes may vary as desired.

Longitudinal movement of the puller wire 42 relative to the catheter body 12, which results in deflection of the tip section 12, is accomplished by suitable manipulation of the control handle 16. The distal end of the control handle 16 comprises a piston 54 with a thumb control 56 for manipulating the puller wire 42. The proximal end of the catheter body 12 is connected to the piston 54 by means of a shrink sleeve 28.

The puller wire 42, lead wires 40 and electromagnetic sensor cable 74 extend through the piston 54. The puller wire 42 is anchored to an anchor pin 36, located proximal to the piston 54. The lead wires 40 and electromagnetic sensor cable 74 extend through a first tunnel 58, located near the side of the control handle 16. The electromagnetic sensor cable 74 connects to the circuit board 64 in the proximal end of the control handle 16. Wires 80 connect the circuit board 64 to a computer and imaging monitor (not shown).

Within the piston 54, the puller wire 42 is situated within a transfer tube 27, and the electromagnetic sensor cable 74 and lead wires 40 are situated within another transfer tube 27 to allow longitudinal movement of the wires and cable near the glue joint 53. The guide tube 66 extends through a second tunnel 60 situated near the side of the control handle 16 opposite the anchor pin 36.

In another preferred embodiment constructed in accordance with the present invention, two or more puller wires (not shown) are provided to enhance the ability to manipulate the tip section. In such an embodiment, a second puller wire and a surrounding second compression coil extend through the catheter body and into separate off-axis lumens in the tip section. The lumens of the tip section receiving the puller wires may be in adjacent quadrants. The first puller wire is preferably anchored proximal to the anchor location of the second puller wire. The second puller wire may be anchored to the tip electrode or may be anchored to the wall of the tip section adjacent the distal end of tip section.

The distance between the distal end of the compression coils and the anchor sites of each puller wire in the tip section determines the curvature of the tip section 14 in the direction of the puller wires. For example, an arrangement wherein the two puller wires are anchored at different distances from the distal ends of the compression coils allows a long reach curve in a first plane and a short reach curve in a plane 90° from the first, i.e., a first curve in one plane generally along the axis of the tip section before it is deflected and a second curve distal to the first curve in a plane transverse, and preferably normal to the first plane. The high torque characteristic of the catheter tip section 12 reduces the tendency for the deflection in one direction to deform the deflection in the other direction.

As an alternative to the above described embodiment, the puller wires (not shown) may extend into diametrically opposed off-axis lumens in the tip section. In such an embodiment,each of the puller wires may be anchored at the same location along the length of the tip section, in which case the curvatures of the tip section in opposing directions are the same and the tip section can be made to deflect in either direction without rotation of the catheter body.

The preceding description has been presented with reference to presently preferred embodiments of the invention. Workers skilled in the art and technology to which this invention pertains will appreciate that alterations and changes in the described structure may be practiced without meaningful departing from the principal, scope of this invention as defined by the accompanying claims.

Accordingly, the foregoing description should not be read as pertaining only to the precise structures described and illustrated in the accompanying drawings, but rather should be read consistent with and as support to the following claims which are to have their fullest and fair scope.

## Claims

1. A steerable cardiac drug injection catheter (10) comprising:
a catheter body (12) having an outer wall (22), proximal and distal ends, and at least one lumen (18) extending therethrough;
a deflection control handle (16) fixedly attached to the proximal end of the catheter body;
a tip section (14) comprising flexible tubing (19) having proximal and distal ends and at least one lumen (30; 32; 34) extending therethrough, the proximal end of the tip section being fixedly attached to the distal end of the catheter body;
a tip electrode (36) mounted at the distal end of the tip section (14), said tip electrode having a distal face and a lumen therethrough and being adapted to measure electrical potentials;
an electrode conductor (40) electrically connected to the tip electrode (36), said conductor extending through the at least one lumen (34) in the tip section (14), through the at least one lumen (18) in the catheter body (12) and into the deflection control handle (16);
an injection needle (46) extending through the at least one lumen (18) in the catheter body (12) and the at least one lumen (30) in the tip section (14) and through the lumen of the tip electrode (36), said needle (46) being slidable from a first position in which the distal tip of the needle is withdrawn into the distal face of the tip electrode (36) to a second position in which the distal tip of the needle extends out of the distal face of the tip electrode;
a needle control handle (17) having an outer body (21) with a lumen (25) and including a slidable needle control knob (23) attached to a piston (23a) having a lumen, the piston being slidably mounted within the lumen (25) in the outer body (21) of the needle control handle, the injection needle (46) being fixedly attached to the outer body (21) and extending through the lumen (25) within the outer body and through the lumen in the piston (23a), the needle control handle arrangement being for sliding the needle (46) from the first position to the second position; and
means (56) for deflecting the distal tip of the catheter (10) upon manipulation of the deflection control handle (16).

2. An injection catheter according to claim 1, further comprising an electromagnetic mapping sensor (72) disposed in the distal portion of the tip section (14) for producing electrical signals indicative of the position of the electromagnetic mapping sensor relative the position of a reference electrode sensor.

3. An injection catheter according to claim 2, further comprising a sensor cable (74) electrically attached to the electromagnetic mapping sensor (72) and extending through the at least one lumen (34) in the tip section (14), through the at least one lumen (18) in the catheter body (12) and into the deflection control handle (16), and the sensor cable (74) is electrically attached to a circuit board (64) situated within the deflection control handle (16).

4. An injection catheter according to claim 3, wherein the deflection control handle (16) comprises a first member (54) fixedly attached to the proximal end of the catheter body (12) and a second member that is movable relative to the first member.

5. An injection catheter according to claim 4, wherein the deflecting means comprises a puller wire (42) having a proximal end and a distal end, the puller wire extending from the deflection control handle (16), through the at least one lumen (18) in the catheter body (12) and is fixedly secured within the tip section (14), and the proximal end of the puller wire (42) is fixedly secured to the second member of the deflection control handle (16), whereby manipulation of the first member (54) of the deflection control handle relative to the second member of the deflection control handle moves the puller wire relative to the catheter body resulting in deflection of the tip section (14).

6. An injection catheter as defined in claim 5, wherein the first member (54) of the deflection control handle (16) is movable from a first position to a second position relative to the second member of the deflection control handle, and the puller wire (42) is fixedly secured to the second member of the deflection control handle such that when the second member of the deflection control handle is moved from the first position to the second position the puller wire (42) causes the tip section (14) of the catheter to be deflected from a normally straight position to a deflected position.

7. An injection catheter according to claim 6, wherein the deflecting control (56) further comprises a compression coil (44) situated in the catheter body (12) in surrounding relation to the puller wire (42) and extending into the at least one lumen (34) in the tip section (14).

8. An injection catheter according to claim 7, wherein the compression coil (44) is anchored to the catheter (10) at the proximal end of the catheter body (12) and at the proximal end of the tip section (14).

9. An injection catheter as defined in claim 1, wherein the outer diameter of the needle (46) is substantially the same as the diameter of the at least one lumen (30) through the tip section (14) to thereby prevent the flow of blood through the lumen (30) of the tip section and the at least one lumen (18) of the catheter body (12).

## Patentansprüche

1. Ein lenkbarer Arzneimittelinjektionsherzkatheter (10), umfassend:
einen Katheterkörper (12) mit einer äußeren Wand (22), einem proximalen und distalen Ende und zumindest einem Lumen (18), das sich durch diesen erstreckt;
einen Ablenkungssteuerungsgriff (16), der fest am proximalen Ende des Katheterkörpers befestigt ist;
einen Spitzenabschnitt (14), der ein flexibles Rohr (19) umfaßt, das ein proximales und distales Ende und zumindest ein Lumen (30; 32; 34) aufweist, das sich durch diesen erstreckt, wobei das proximale Ende des Spitzenabschnitts fest am distalen Ende des Katheterkörpers befestigt ist;
eine Punktelektrode (36), die am distalen Ende des Spitzenabschnitts (14) montiert ist, wobei die Punktelektrode eine distale Fläche und ein Lumen durch diese aufweist und dafür eingerichtet ist, elektrische Potentiale zu messen;
einen Elektrodenleiter (40), der elektrisch mit der Punktelektrode (36) verbunden ist, wobei sich der Leiter durch das zumindest eine Lumen (34) im Spitzenabschnitt (14) durch das zumindest eine Lumen (18) im Katheterkörper (12) und in den Ablenkungssteuerungsgriff (16) erstreckt;
eine Injektionsnadel (46), die sich durch das zumindest eine Lumen (18) im Katheterkörper (12) und das zumindest eine Lumen (30) im Spitzenabschnitt (14) und durch das Lumen der Punktelektrode (36) erstreckt, wobei die Nadel (46) von einer ersten Position, in der die distale Spitze der Nadel in die distale Fläche der Punktelektrode (36) zurückgezogen ist, zu einer zweiten Position verschiebbar ist, in der die distale Spitze der Nadel sich aus der distalen Fläche der Punktelektrode erstreckt;
einen Nadelsteuerungsgriff (17) mit einem äußeren Körper (21) mit einem Lumen (25) und welcher einen verschiebbaren Nadelsteuerungsknopf (23) aufweist, der an einem Kolben (23a) befestigt ist, der ein Lumen aufweist, wobei der Kolben verschiebbar im Lumen (25) im äußeren Körper (21) des Nadelsteuerungsgriffs montiert ist, wobei die Injektionsnadel (46) fest am äußeren Körper (21) befestigt ist und sich durch das Lumen (25) im äußeren Körper und durch das Lumen im Kolben (23a) erstreckt, wobei die Nadelsteuerungsgriffanordnung für ein Verschieben der Nadel (46) von der ersten Position in die zweite Position vorgesehen ist; und
Mittel (56) zum Ablenken der distalen Spitze des Katheters (10) bei einer Handhabung des Ablenkungssteuerungsgriffs (16).

2. Injektionskatheter nach Anspruch 1, welcher des weiteren eine elektromagnetischen Abbildungssensor (72) aufweist, der im distalen Teil des Spitzenabschnitts (14) angeordnet ist, zum Erzeugen elektrischer Signale, die für die Position des elektromagnetischen Abbildungssensors im Verhältnis zur Position eines Referenzelektrodensensors kennzeichnend sind.

3. Injektionskatheter nach Anspruch 2, der des weiteren ein Sensorkabel (74) aufweist, das elektrisch am elektromagnetischen Abbildungssensor (72) befestigt ist und sich durch das zumindest eine Lumen (34) im Spitzenabschnitt (14), durch das zumindest eine Lumen (18) im Katheterkörper (12) und in den Ablenkungssteuerungsgriff (16) erstreckt und wobei das Sensorkabel (74) elektrisch an einer Schaltungsplatine (46) befestigt ist, die sich im Ablenkungssteuerungsgriff (16) befindet.

4. Injektionskatheter nach Anspruch 3, wobei der Ablenkungssteuerungsgriff (16) ein erstes Element (54), das fest am proximalen Ende des Katheterkörpers (12) befestigt ist, und ein zweites Element aufweist, das relativ zum ersten Element beweglich ist.

5. Injektionskatheter nach Anspruch 4, wobei das Ablenkungsmittel einen Zugdraht (42) mit einem proximalen Ende und einem distalen Ende umfaßt, sich der Zugdraht vom Ablenkungssteuerungsgriff (16) durch das zumindest eine Lumen (18) in den Katheterkörper (12) erstreckt und fest im Spitzenabschnitt (14) gesichert ist und das proximale Ende des Zugdrahts (42) fest am zweiten Element des Ablenkungssteuerungsgriffs (16) gesichert ist, wobei eine Handhabung des ersten Elements (54) des Ablenkungssteuerungsgriffs im Verhältnis zum zweiten Element des Ablenkungssteuerungsgriffs den Zugdraht im Verhältnis zum Katheterkörper bewegt, was zu einer Ablenkung des Spitzenabschnitts (14) führt.

6. Injektionskatheter nach Anspruch 5, wobei das erste Element (54) des Ablenkungssteuerungsgriffs (16) im Verhältnis zum zweiten Element des Ablenkungssteuerungsgriffs von einer ersten Position zu einer zweiten Position bewegbar ist und der Zugdraht (42) fest am zweiten Element des Ablenkungssteuerungsgriffs gesichert ist, so daß, wenn das zweite Element des Ablenkungssteuerungsgriffs von der ersten Position zur zweiten Position bewegt wird, der Zugdraht (42) bewirkt, daß der Spitzenabschnitt (14) des Katheters von einer normalerweise geraden Position zu einer abgelenkten Position abgelenkt wird.

7. Injektionskatheter nach Anspruch 6, wobei die Ablenkungssteuerung (56) des weiteren eine Kompressionsspule (44) aufweist, die sich im Katheterkörper (12) in einer umgebenden Beziehung zum Zugdraht (42) befindet und sich in das zumindest eine Lumen (34) im Spitzenabschnitt (14) erstreckt.

8. Injektionskatheter nach Anspruch 7, wobei die Kompressionsspule (44) am Katheter (10) am proximalen Ende des Katheterkörpers (12) und am proximalen Ende des Spitzenabschnitts (14) verankert ist.

9. Injektionskatheter nach Anspruch 1, wobei der äußere Durchmesser der Nadel (46) im wesentlichen der gleiche ist wie der Durchmesser des zumindest einen Lumen (30) durch den Spitzenabschnitt (14), um dadurch den Blutfluß durch das Lumen (30) des Spitzenabschnitts und das zumindest eine Lumen (18) des Katheterkörpers (12) zu verhindern.

## Revendications

1. Cathéter (10) d'injection de médicament cardiaque orientable comprenant :
un corps de cathéter (12) présentant une paroi extérieure (22), des extrémités proximales et distales, et au moins une lumière (18) qui s'étend à travers ;
une poignée de commande de déviation (16) attachée de manière fixe à l'extrémité proximale du corps de cathéter ;
une partie de bout (14) comprenant un tuyau souple (19) ayant des extrémités proximales et distales et au moins une lumière (30 ; 32 ; 34) s'étendant à travers, l'extrémité proximale de la partie de bout étant attachée de manière fixe à l'extrémité distale du corps de cathéter ;
une électrode de bout (36) montée au niveau de l'extrémité distale de la partie de bout (14), ladite électrode de bout ayant une face distale et une lumière à travers et étant adaptée pour mesurer des potentiels électriques ;
un conducteur d'électrode (40) connecté de manière électrique à l'électrode de bout (36), ledit conducteur s'étendant à travers l'au moins une lumière (34) dans la partie de bout (14), à travers l'au moins une lumière (18) dans le corps de cathéter (12) et dans la poignée de commande de déviation (16) ;
une aiguille d'injection (46) s'étendant à travers l'au moins une lumière (18) dans le corps de cathéter (12) et l'au moins une lumière (30) dans la partie de bout (14) et à travers la lumière de l'électrode de bout (36), ladite aiguille (46) pouvant coulisser à partir d'une première position dans laquelle le bout distal de l'aiguille est retiré dans la face distale de l'électrode de bout (36), vers une seconde position dans laquelle le bout distal de l'aiguille s'étend hors de la face distale de l'électrode de bout ;
une poignée de commande d'aiguille (17) ayant un corps extérieur (21) avec une lumière (25) et comprenant un bouton de commande d'aiguille pouvant coulisser (23) attaché à un piston (23a) ayant une lumière, le piston étant monté de façon coulissante à l'intérieur de la lumière (25) dans le corps extérieur (21) de la poignée de commande d'aiguille, l'aiguille d'injection (46) étant attachée de manière fixe au corps extérieur (21) et s'étendant à travers la lumière (25) à l'intérieur du corps extérieur et à travers la lumière dans le piston (23a), l'agencement de poignée de commande d'aiguille servant à faire coulisser l'aiguille (46) à partir de la première position vers la seconde position ; et
des moyens (56) pour faire dévier le bout distal du cathéter (10) lors de la manipulation de la poignée de commande de déviation (16).

2. Cathéter d'injection selon la revendication 1, comprenant de plus un capteur de cartographie électromagnétique (72) disposé dans la partie distale de la partie de bout (14) pour produire des signaux électriques indicatifs de la position du capteur de cartographie électromagnétique par rapport à la position d'un capteur à électrode de référence.

3. Cathéter d'injection selon la revendication 2, comprenant de plus un câble de détecteur (74) attaché de manière électrique au capteur de cartographie électromagnétique (72) et s'étendant à travers l'au moins une lumière (34) dans la partie de bout (14), à travers l'au moins une lumière (18) dans le corps de cathéter (12) et dans la poignée de commande de déviation (16), et le câble de capteur (74) est attaché de manière électrique à une carte de circuit (64) située à l'intérieur de la poignée de commande de déviation (16).

4. Cathéter d'injection selon la revendication 3, dans lequel la poignée de commande de déviation (16) comprend un premier élément (54) attaché de manière fixe à l'extrémité proximale du corps de cathéter (12) et un second élément qui est mobile par rapport au premier élément.

5. Cathéter d'injection selon la revendication 4, dans lequel les moyens de déviation comprennent un câble de tirage (42) ayant une extrémité proximale et une extrémité distale, le câble de tirage s'étendant à partir de la poignée de commande de déviation (16), à travers l'au moins une lumière (18) dans le corps de cathéter (12) et est fixé de manière fixe à l'intérieur de la partie de bout (14), et l'extrémité proximale du câble de tirage (42) est fixée de manière fixe au second élément de la poignée de commande de déviation (16), grâce à quoi la manipulation du premier élément (54) de la poignée de commande de déviation par rapport au second élément de la poignée de commande de déviation déplace le câble de tirage par rapport au corps de cathéter ce qui se traduit par une déviation de la partie de bout (14).

6. Cathéter d'injection selon la revendication 5, dans lequel le premier élément (54) de la poignée de commande de déviation (16) est mobile à partir d'une première position vers une seconde position par rapport au second élément de la poignée de commande de déviation, et le câble de tirage (42) est fixé de manière fixe au second élément de la poignée de commande de déviation de telle sorte que lorsque le second élément de la poignée de commande de déviation est déplacé à partir de la première position jusqu'à la seconde position, le câble de tirage (42) provoque la déviation de la partie de bout (14) du cathéter à partir d'une position normalement droite vers une position déviée.

7. Cathéter d'injection selon la revendication 6, dans lequel la commande de déviation (56) comprend de plus un ressort à boudin de compression (44) situé dans le corps de cathéter (12) en entourant le câble de tirage (42) et en s'étendant dans l'au moins une lumière (34) dans la partie de bout (14).

8. Cathéter d'injection selon la revendication 7, dans lequel le ressort boudin de compression (44) est ancré au cathéter (10) au niveau de l'extrémité proximale du corps de cathéter (12) et au niveau de l'extrémité proximale de la partie de bout (14).

9. Cathéter d'injection selon la revendication 1, dans lequel le diamètre extérieur de l'aiguille (46) est sensiblement identique au diamètre de l'au moins une lumière (30) à travers la partie de bout (14) pour éviter de ce fait l'écoulement du sang à travers la lumière (30) de la partie de bout et l'au moins une lumière (18) du corps de cathéter (12).
